# EUROPEAN PATENT SPECIFICATION

(11) **EP 3 013 976 B1**
(45) Date of publication and mention of the grant of the patent: **02.01.2019**
(21) Application number: 14742257.0
(22) Date of filing: 25.06.2014
(51) Int. Cl.: C12Q 1/6883

(54) **METHOD OF PREDICTING RISK FOR TYPE 1 DIABETES BEFORE SEROCONVERSION**
VERFAHREN ZUR VORHERSAGE DES RISIKOS VON TYP-1-DIABETES VOR EINER SEROKONVERSION
PROCÉDÉ DE PRÉDICTION DU RISQUE DE DIABÈTE DE TYPE 1 AVANT SEROCONVERSION

(30) Priority: 25.06.2013 US 201361839123 P
(43) Date of publication of application: 04.05.2016
(73) Proprietor: Turun Yliopisto, 20014 Turun yliopisto (FI)
(72) Inventor: KALLIONPÄÄ, Henna, FI-20740 Turku (FI); ELO, Laura L., FI-20720 Turku (FI); LAAJALA, Essi, FI-20700 Turku (FI); MYKKÄNEN, Juha, FI-20540 Turku (FI); SIMELL, Olli, FI-21260 Raisio (FI); LAHESMAA, Riitta, FI-20880 Turku (FI); LÄHDESMÄKI, Harri, FI-02130 Espoo (FI); KONG, Lingjia, 20014 Turun Yliopisto (FI); TUOMELA, Soile, 20014 Turun Yliopisto (FI); LIETZÉN, Niina, 20014 Turun Yliopisto (FI)
(74) Representative: Kolster Oy Ab
(86) International application number: PCT/FI2014/050522
(87) International publication number: WO 2014/207312

(56) References cited:
- EP-A1- 1 840 573
- US-A1- 2012 329 071
- ESSI LAAJALA: "Type 1 diabetes biomarkers in human hole blood transcriptome", INTERNET CITATION, 24 June 2013 (2013-06-24), pages 1-23, XP002731162, Retrieved from the Internet: URL:http://www.utu.fi/en/units/sci/units/m ath/Research/optimization/Documents/sovmat 20042012.pdf [retrieved on 2014-10-14]
- KALLIONPÄÄ HENNA ET AL: "Innate immune activity is detected prior to seroconversion in children with HLA-conferred type 1 diabetes susceptibility", DIABETES, AMERICAN DIABETES ASSOCIATION, US, vol. 63, no. 7, 1 July 2014 (2014-07-01), pages 2402-2414, XP009180736, ISSN: 1939-327X, DOI: 10.2337/DB13-1775
- FERREIRA RICARDO C ET AL: "A type I interferon transcriptional signature precedes autoimmunity in children genetically at risk for type 1 diabetes", DIABETES, AMERICAN DIABETES ASSOCIATION, US, vol. 63, no. 7, 1 July 2014 (2014-07-01), pages 2538-2550, XP009180749, ISSN: 0012-1797, DOI: 10.2337/DB13-1777 [retrieved on 2014-02-21]
- Essi Laajala: "Type 1 diabetes biomarkers in human whole blood transcriptome", , 24 June 2013 (2013-06-24), pages 1-23, XP002731162, Retrieved from the Internet: URL:http://www.utu.fi/en/units/sci/units/m ath/Research/optimization/Documents/sovmat 20042012.pdf [retrieved on 2014-10-14]
- KALLIONPÄÄ HENNA ET AL: "Innate immune activity is detected prior to seroconversion in children with HLA-conferred type 1 diabetes susceptibility", DIABETES, US, vol. 63, no. 7, 18 February 2014 (2014-02-18), pages 2402-2414, XP009180736, ISSN: 1939-327X
- FERREIRA RICARDO C ET AL: "A type I interferon transcriptional signature precedes autoimmunity in children genetically at risk for type 1 diabetes", DIABETES, AMERICAN DIABETES ASSOCIATION, US, vol. 63, no. 7, 21 February 2014 (2014-02-21), pages 2538-2550, XP009180749, ISSN: 0012-1797 [retrieved on 2014-02-21]
- ESSI LAAJALA: "Type 1 diabetes biomarkers in human hole blood transcriptome", INTERNET CITATION, 24 June 2013 (2013-06-24), pages 1-23, XP002731162, Retrieved from the Internet: URL:http://www.utu.fi/en/units/sci/units/m ath/Research/optimization/Documents/sovmat 20042012.pdf [retrieved on 2014-10-14]
- KALLIONPÄÄ HENNA ET AL: "Innate immune activity is detected prior to seroconversion in children with HLA-conferred type 1 diabetes susceptibility", DIABETES, AMERICAN DIABETES ASSOCIATION, US, vol. 63, no. 7, 1 July 2014 (2014-07-01), pages 2402-2414, XP009180736, ISSN: 1939-327X, DOI: 10.2337/DB13-1775
- FERREIRA RICARDO C ET AL: "A type I interferon transcriptional signature precedes autoimmunity in children genetically at risk for type 1 diabetes", DIABETES, AMERICAN DIABETES ASSOCIATION, US, vol. 63, no. 7, 1 July 2014 (2014-07-01), pages 2538-2550, XP009180749, ISSN: 0012-1797, DOI: 10.2337/DB13-1777 [retrieved on 2014-02-21]

## Description

### FIELD OF THE INVENTION

The present invention relates to the field of molecular diagnostics. More specifically the present invention relates to means and methods for predicting, before seroconversion, a risk of a subject for Type 1 diabetes (T1D).

### BACKGROUND OF THE INVENTION

By the time of clinical diagnosis of Type 1 diabetes (T1D), an estimated 80-90% of insulin producing pancreatic beta cells have been destroyed by autoimmune mechanisms with immune cells infiltrating the islets. Autoantibodies against beta cell-specific antigens, such as proinsulin and insulin, glutamic acid decarboxylase 65 (GAD-65), islet antigen-2 (IA-2), and zinc transporter 8 (ZnT8), can usually be detected in serum before the clinical onset of the disease, but although the number of circulating autoantibodies is predictive for the development of T1D, predicting the onset of clinical disease remains a challenge.

Susceptibility to T1D is inherited, with the human leukocyte antigen (HLA) class II haplotypes encoding DR3-DQ2 *(DRB1*03-DQA1*0501-DQB1*0201)* and DR4-DQ8 *(DRB1*0401-DQA1*0301-DQB1*0302)* molecules conferring an estimated 50% of the total genetic risk. In addition, more than 40 non-HLA genes with more modest effect on disease risk, such as *INS, CTLA4, PTPN22,* and *IL2R,* have been discovered. When combined with a suitable genetic background, largely unknown random environmental factors probably contribute to the breakdown of self-tolerance. For instance, dietary factors, such as early exposure to foreign complex proteins and vitamin D deficiency, have been associated with T1D pathogenesis, and viral infections are believed to trigger autoimmunity. T1D prevalence is also associated with westernized lifestyle and level of hygiene. ESSI LAAJALA: "Type 1 diabetes biomarkers in human hole blood transcriptome", 24 June 2013, URL:http://www.utu.fi/en/units/ sci/units/math/Research/optimization/Documents/sovmat20042012.pdf, discloses differentially expressed genes in Type 1 diabetes before seroconversion and their potential in prognosing Type 1 diabetes.

There is a need for kits and methods for predicting the risk of development of T1D prior to any signs of seroconversion.

### BRIEF DESCRIPTION OF THE INVENTION

The present invention is defined in the appended claims. In one aspect the present disclosure provides a method of predicting, before seroconversion, a risk of a subject for Type 1 diabetes (T1D). The method may comprise the steps of a) providing a sample obtained from said subject, b) determining a gene expression profile of at least C17orf97 in said sample, and c) using said gene expression profile for the prediction of the probability of the subject to develop T1D.

In some embodiments, said method further comprises determining a gene expression profile of at least one further gene selected from the group consisting of ERAP2, PMP22, VNN1, PRSS33, and NPRL3.

In some further embodiments, said method further comprises determining a gene expression profile of at least one further gene selected from the group consisting of SLC29A1, SUB1, EMR3, DCAF1, AHI1, SETD9, CREB5, TMEM158, ROPN1L, OLIG2, HIST1H4A (includes others), PROK2, GPR84, C19orf59, CEACAM1, SCOC, CACNG6, IRF5, HOXB2, CLC, C5orf4, TNS1, SLC22A4, PRSS23, PHOSPHO1, RPS27L, SNCA, IFIT3, KRT1, VWCE, BCL6, EPB49, DTL, HBD, ASGR2, LILRB3, RSAD2, TMEM126B, ANXA3, GLT25D2, MYOF, IFI27, CENPA, KLRD1, ITGB1BP1, RSL24D1, GBP5, TGM3, RAB31, HLA-C, NFIX, COX7B, OAS1, IL18RAP, CYP4F3, LRG1, SLC6A8, IFITM3, CEP55, EPB42, C9orf123, MX1, MMP9, IGF2BP2, MBNL3, C3AR1, HLA-DPB1, TRIM58, FAM46C, CTTN, RBPMS2, IFI44L, C4BPA, CPPED1, SLC4A1, GNLY, GMPR, CASP5, ADM, SMOX, CNN2, CYSTM1, COMMD6, HINT1, DSC2, SORT1, SIRPB1, SEC14L1, RRP12, OAS3, SELENBP1, PTER, RFX2, RNASE2, TSHZ3, VCAN, DEFA1, SAMD9L, MAFB, KIAA1324, OSBP2, HMMR, IFIT2, CBS, FOLR3, CCL4L1/CCL4L2, XAF1, NPCDR1, FAM129A, KLRF1, IFI44, PI3, CCL8, EPSTI1, ALPL, HERC5, DYSF, RPL26.

In another aspect, the present disclosure provides an *in vitro* screening kit for determining, prior to seroconversion, whether an individual, preferably having an HLA-conferred risk, is at risk of developing T1D. The kit may comprise one or more testing agents capable of detecting the expression level of at least C17orf97 and, optionally, one or more genes selected from any gene profile disclosed hereinabove, in a biological sample obtained from a subject whose risk of developing T1D is to be determined.

Other aspects, specific embodiments, objects, details, and advantages of the disclosure are set forth in the following drawings, detailed description, examples, and dependent claims.

### BRIEF DESCRIPTION OF THE DRAWINGS

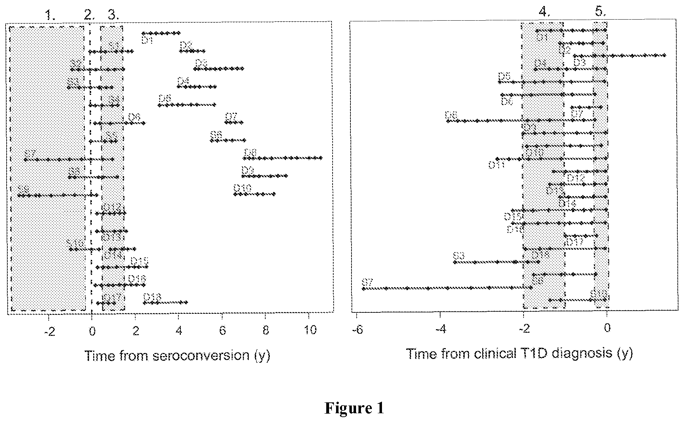

In the following the invention will be described in greater detail by means of preferred embodiments with reference to the attached [accompanying] drawings, in which
Figure 1 illustrates timing of blood sample collection showing time-windows used for temporal analysis of the data. (A) Sample series of seroconverted cases (S, marked with green color) and T1D progressors (D, in red color) relative to the time from seroconversion (in years). Each black diamond represents a blood sample that was subsequently analyzed by transcriptional profiling. For the analysis of temporal changes in gene expression, samples representing three time-windows relative to seroconversion were selected as follows: 1) before seroconversion (22 samples from 6 case-control pairs), 2) at the time of the seroconversion (8 samples from 8 pairs), and 3) 6-18 months after seroconversion (40 samples from 14 pairs). (B) Sample series of seroconverted cases (S, green color) and T1D progressors (D, red color) relative to the time from clinical T1D diagnosis (in years). Samples representing two time-windows relative to T1D diagnosis were selected as follows: 4) 1-2 years before clinical diagnosis (48 samples from 19 pairs), and 5) at the time of clinical diagnosis (18 samples from 18 pairs). (A-B) The time-windows were based on the maximal representation of the samples. See Table 4 for more details on study subjects.

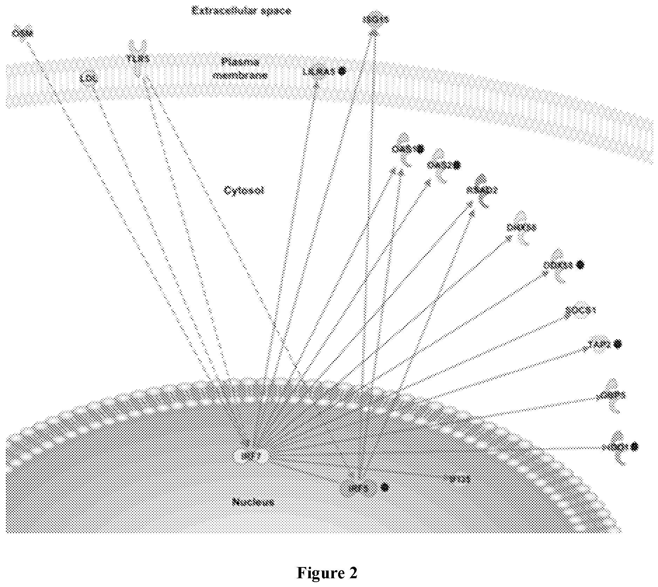

Figure 2 demonstrates transcriptional regulation during T1D pathogenesis. Interferon regulatory factors 5 and 7 (IRF5 and IRF7), the most highly regulated transcription factors in the seroconverted cases, and their central role in the seroconversion-specific transcription. The network was generated with IPA network neighborhood tool, and it shows all the IRF5 and 7-connected genes (nodes) that were differentially regulated in the seroconverters (FDR<0.05). The genes are organized according to their cellular localization. The genes regulating IRF5 or 7 are presented on the left, and the downstream target genes of IRF5 or 7 on the right side. Solid edge indicates direct interaction, dashed edge indirect interaction. Protein-protein interactions are marked with blue color, transcriptional induction/reduction with green, promoter binding with orange and mixed interactions with red. Black dots indicate the genes having a *cis* eQTL effect with SNPs residing in a window of +- 250 kilobases around the gene coordinates (data not shown).

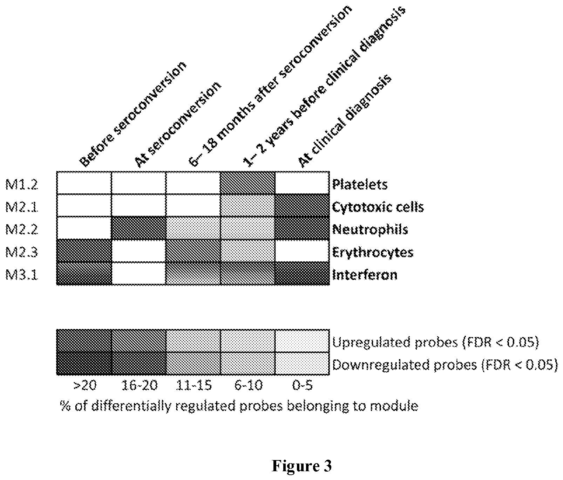

Figure 3 shows a summary of the functional modules regulated in the distinct phases of T1D development. Up-regulation or down-regulation of the module is marked with red or blue, respectively, and the color intensity indicates the percentage of the regulated genes belonging to that module.

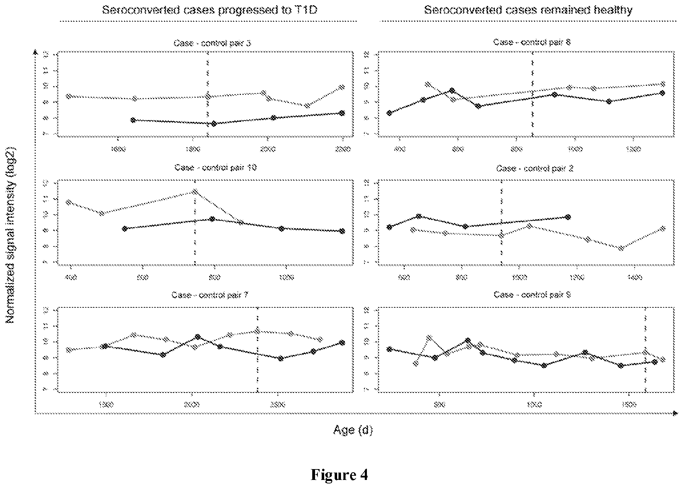

Figure 4 demonstrates that ribonuclease RNase A family 2 (*RNASE2*) gene is up-regulated in such seroconverted cases who later progress to clinical T1D. Longitudinal expression intensity profiles for *RNASE2* (probe set 11729772_at on Affymetrix U219 array) in seroconverted cases (in orange) and their matched autoantibody-negative controls (in black) are presented. Seroconverted cases 3, 7 and 10 later progressed to clinical T1D (20, 22 and 1 month after the last follow-up sample, respectively), whereas seroconverted cases 2, 8 and 9 have remained healthy to-date. The x-axis represents the age of the children (in days) and the y-axis the normalized logarithmic signals on the arrays. The time of seroconversion for the case is marked with a dashed line. Profiles for children with several measurements before seroconversion are presented (see Fig. 1 and Table 4 for more details).

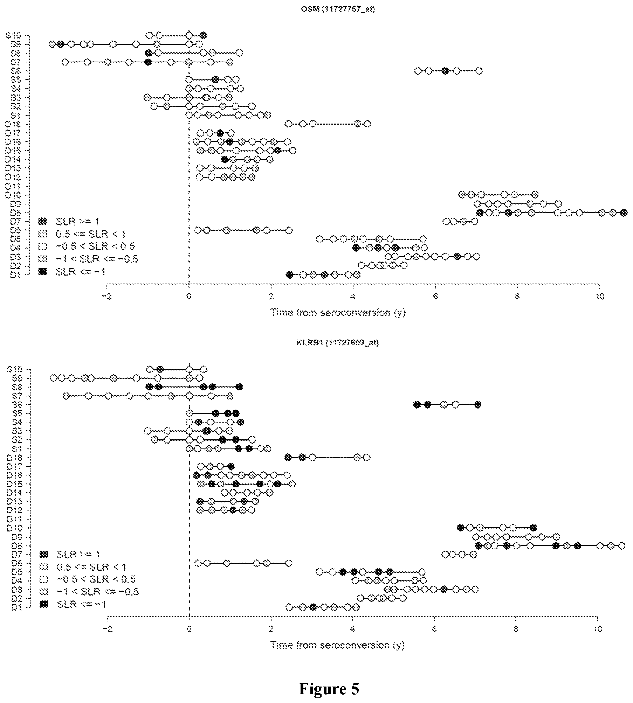

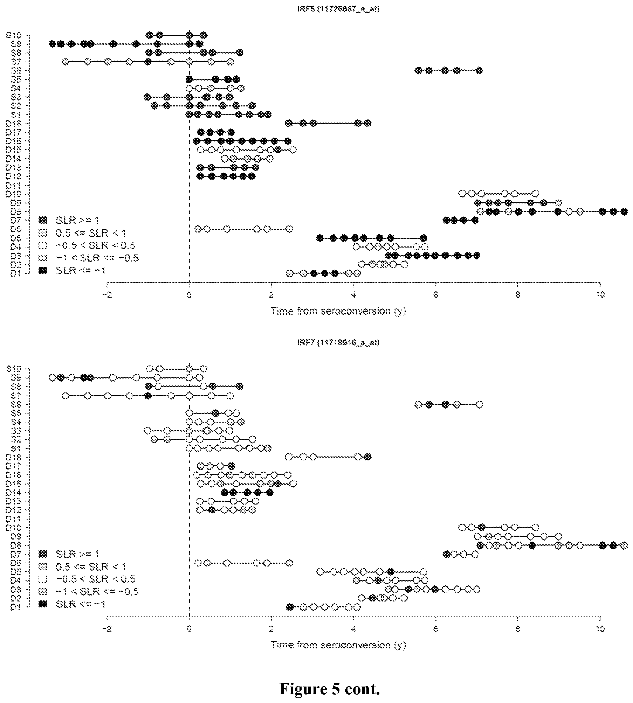

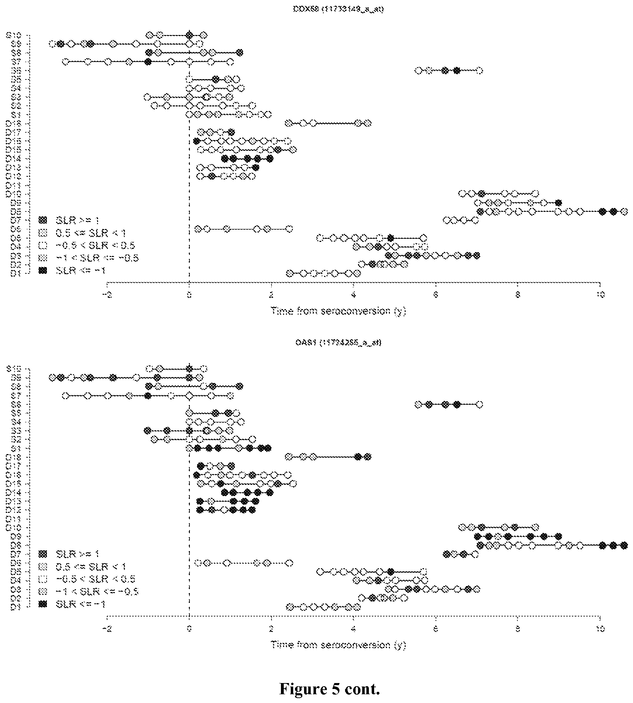

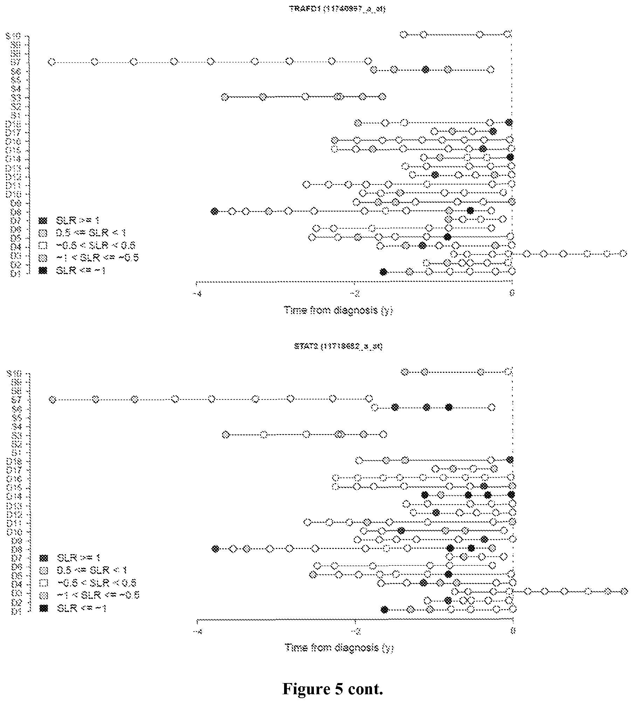

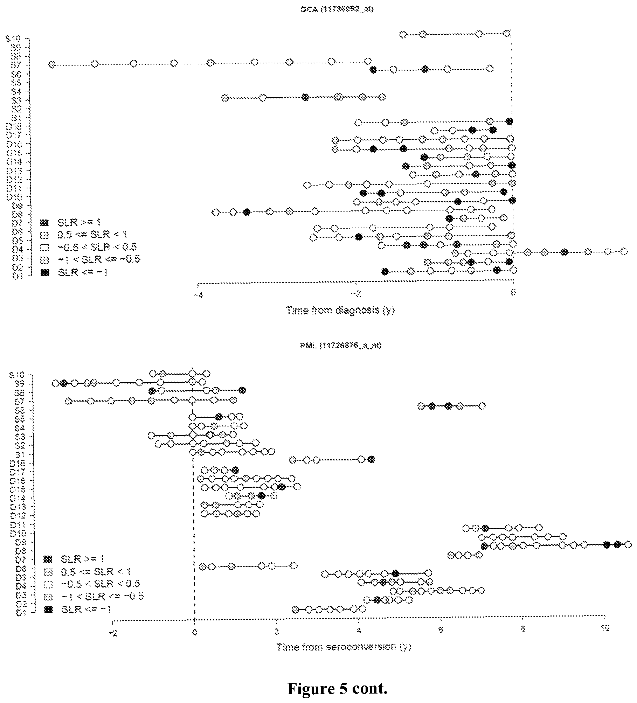

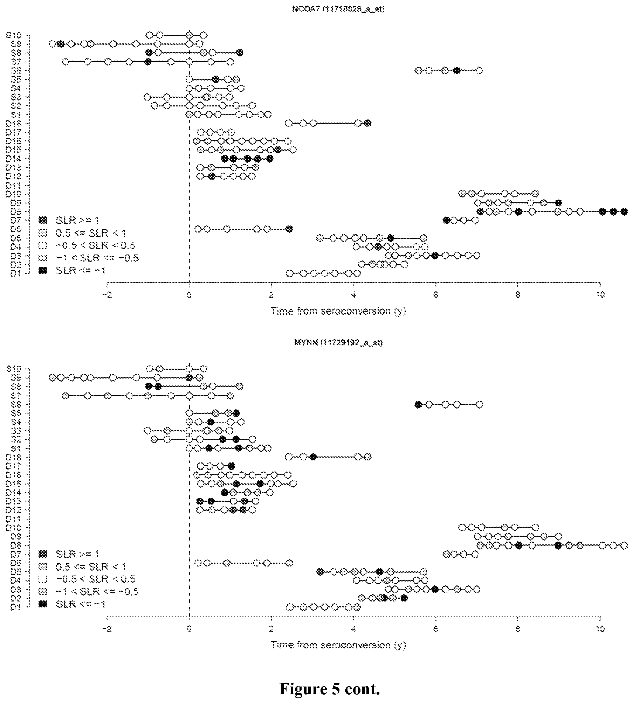

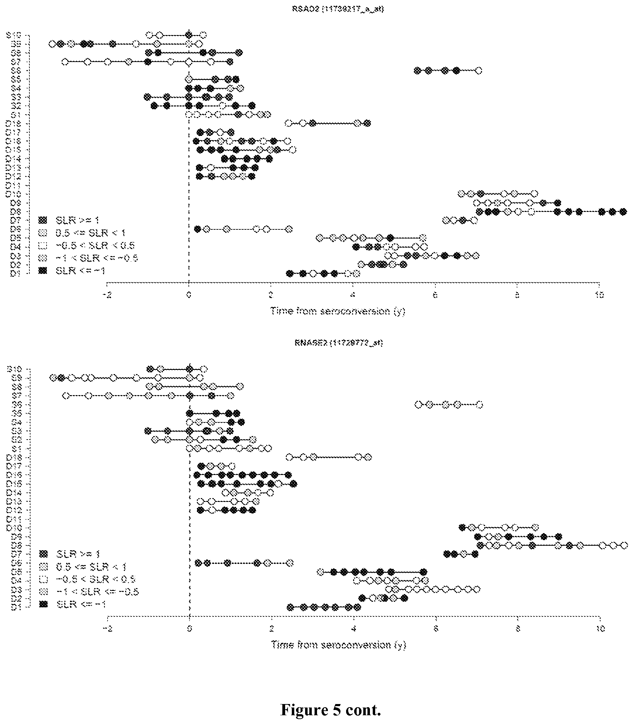

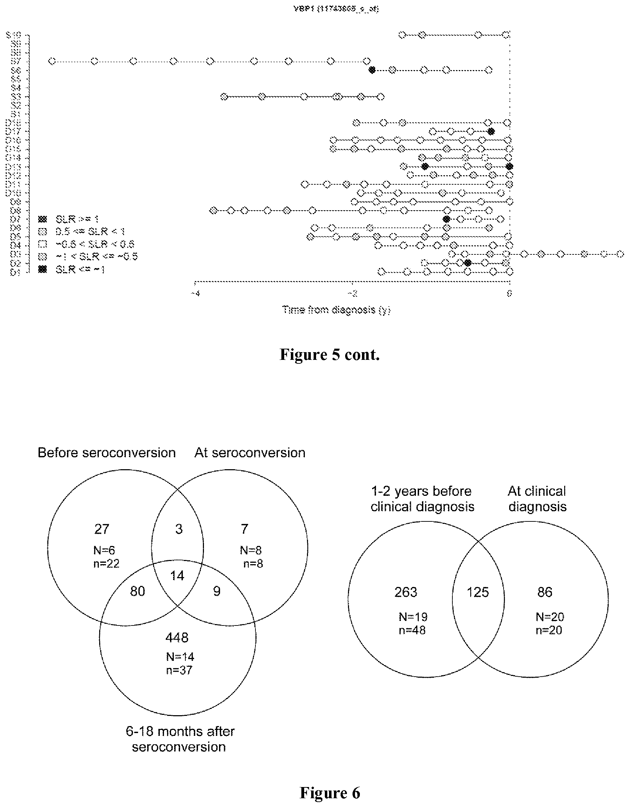

Figure 5 shows time profiles of the differentially regulated genes described in the manuscript relative to the time of seroconversion or clinical diagnosis. Each open circle represents a blood sample of the case that was subsequently analyzed by transcriptional profiling. S denotes the seroconversion cases and D the T1D progressors (Table 4). The color of the circles represents the signal log ratio (SLR) calculated against the matched control (red = up-regulation, blue = downregulation).
Figure 6 demonstrates transcriptional changes in distinct phases of the T1D autoimmune process. Numbers of regulated genes (FDR < 0.05) are listed for each time-window in the analysis. N = case control pairs in the analysis, n = number of samples in the analysis.

### DETAILED DESCRIPTION OF THE INVENTION

In some implementations, the present disclosure is directed to methods, transcriptome profiles and kits useful for determining, before seroconversion, the risk that an individual will develop Type 1 diabetes (T1D).

As used herein, the term "seroconversion" refers to the first detection of one or several T1D-associated autoantibodies against beta cell-specific antigens in serum. These include islet cell specific autoantibodies (ICA), insulin autoantibodies (IAA), glutamic acid decarboxylase 65 autoantibodies (GADA), islet antigen-2 autoantibodies (IA-2A), and zinc transporter 8 autoantibodies (ZnT8A). In some embodiments, the following cut-off values may be used for determining the presence or absence of the autoantibodies: ICA ≥ 4 JDFU (Juvenile Diabetes Foundation units), IAA ≥ 3.48 RU (relative units), GADA ≥ 5.36 RU, IA-2A ≥ 0.43 RU, and ZnT8A ≥ 0.61 RU. Seroconversion may occur years, e.g. 1 to 2 years, before clinical diagnosis.

As used herein, the term "transcriptome profile" refers to the set of RNA molecules, including but not limited to messenger RNA (mRNA) molecules and non-coding RNA (ncRNA) molecules, such as microRNA (miRNA) and long non-coding RNA (long ncRNA, IncRNA), produced in a given sample or a population of cells. It may also refer to a cDNA profile produced from an mRNA profile. The term may be used interchangeably with the terms "gene expression profile", "transcriptional signature", "RNA signature" and the like, as is evident to a person skilled in the art.

As used herein, the terms "up-regulation" and "up-regulated" refer to increase in expression of a gene in a sample as compared to a corresponding control sample. More specifically, the terms refer to increased transcription of a specific mRNA. Said increase can be determined qualitatively and/or quantitatively according to standard methods known in the art. The expression is increased if the expression level of the gene in the sample is, for instance, at least about 1.5 times, 1.75 times, 2 times, 3 times, 4 times, 5 times, 6 times, 8 times, 9 times, time times, 10 times, 20 times or 30 times the expression level of the same gene in the control sample.

As used herein, the terms "down-regulation" and "down-regulated" refer to decrease in expression of a gene in a sample as compared to a corresponding control sample. More specifically, the terms refer to decreased transcription of a specific mRNA. Said decrease can be determined qualitatively and/or quatitatively according to standard methods known in the art. The expression is decreased if the expression level of the gene in the sample is, for instance, at least about 1.5 times, 1.75 times, 2 times, 3 times, 4 times, 5 times, 6 times, 8 times, 9 times, time times, 10 times, 20 times or 30 times lower than the expression level of the same gene in the control sample.

As used herein, the term "updown-regulated" refers to three alternative situations: i) said gene is up-regulated in some subjects while down-regulated in other subjects; ii) some alternative probes for a given gene show up-regulation while some other probes for the same gene show down-regulation; and iii) before seroconversion, said gene shows both up-regulation and down-regulation de-pending on the measurement time point used.

The inventors have herein identified a transcriptome profile which exhibits predictive value regarding an individual's risk of developing T1D. Importantly, said prediction can be made prior to any signs of seroconversion. The identified predictive transcriptome profile applies in particular to individuals having a Human Leukocyte Antigen (HLA) -conferred risk for T1D. As used herein, the term "HLA-conferred risk for T1D" refers to a predisposition to T1D as determined on the basis of the individual's HLA profile as generally known in the art. In some embodiments, HLA-conferred susceptibility takes place if the individual carries HLA-DQB1 alleles *02/*0302 or *0302. If the individual whose risk for T1D is to be determined has HLA-conferred susceptibility, then also the control gene profile should be obtained from an individual having a corresponding HLA-conferred susceptibility or from a pool of such individuals.

As described in the experimental part, the present disclosure is based on a genome-wide transcriptomics analysis on a unique series of prospective whole-blood RNA samples from at-risk children collected in the Finnish Type 1 Diabetes Prediction and Prevention (DIPP) study. The samples covered the time span from before the development of autoantibodies (seroconversion) through the diagnosis of diabetes. Healthy, persistently autoantibody-negative children matched for date and place of birth, gender and the HLA-DQB1-conferred genetic risk were chosen as controls.

In one aspect, the present disclosure is directed to a method of predicting, before seroconversion, a risk of a subject for Type 1 diabetes (T1D) comprising the following steps:
a) providing a sample from said subject,
b) determining a gene expression profile in said sample, and
c) using said gene expression profile for the prediction of the probability of the subject to develop T1D. Preferably, the determined gene expression profile is compared to that of a corresponding control.

The above aspect may also be defined differently, i.e. as a method of predicting risk of T1D in an individual before seroconversion, the method comprising:
a) measuring the level of one or more members of one or more predictive gene profiles described herein, in a sample obtained from the individual,
b) identifying, preferably by a processor of a computing device, for the one or more measured levels, a difference between the measured level and a corresponding predetermined control level, and
c) responsive to the identifying, determining, preferably by the processor, a prediction corresponding to a relative risk of the individual developing T1D.

Furthermore, the above aspect may also be defined differently, i.e. as a method of predicting risk of T1D in an individual, the method comprising:
a) measuring one or more gene expression levels of any gene profile described herein, in a sample obtained from the individual,
b) calculating, preferably by a processor of a computing device, a risk assessment score corresponding to a relative risk of the individual developing T1D, wherein the risk assessment score is based in part upon a comparison of the gene expression level and a corresponding, possibly predetermined, control level.

In some embodiments, the risk for developing T1D can be determined based on the up-regulation of one or more genes selected from non-interferon regulated genes listed in Table 1 as compared to a corresponding control level. In some embodiments, a predetermined control level may be used.

**Table 1. Non-interferon regulated genes whose up-regulation indicates risk for developing T1D**

| **Gene Symbol** | **Gene Bank ID** | **Gene Name** | **Gene Type** |
|---|---|---|---|
| RNASE2 | 6036 | ribonuclease, RNase A family, 2 (liver, eosinophil-derived neurotoxin) | enzyme |
| CPPED1 | 55313 | calcineurin-like phosphoesterase domain containing 1 | enzyme |
| VNN1 | 8876 | vanin 1 | enzyme |
| ALPL | 249 | alkaline phosphatase, liver/bone/kidney | phosphatase, enzyme |
| PHOSPHO1 | 162466 | phosphatase orphan 1 | enzyme |
| EMR3 | 84658 | EGF-like module containing, mucin-like, hormone receptor-like 3 | G-protein coupled receptor |
| CREB5 | 9586 | cAMP responsive element binding protein 5 | transcription regulator |
| ROPN1L | 83853 | rhophilin associated tail protein 1-like | kinase, enzyme |
| CLC | 1178 | Charcot-Leyden crystal protein | enzyme |
| DEFA1 | 1667 | defensin, alpha 1 | |
| PRSS33 | 260429 | protease, serine 33 | enzyme |

Alternatively or additionally, the risk for developing T1D can be determined based on the up-regulation of one or more genes selected from interferon-regulated genes listed in Table 2 and/or on down-regulation of PMP22 (peripheral myelin protein 22, Gene Bank ID 5376) as compared to a corresponding control level. In some embodiments, a predetermined control level may be used.

**Table 2. Interferon-regulated genes whose up-regulation indicates risk for developing T1D**

| **Gene Symbol** | **Gene Bank ID** | **Gene Name** | **Gene Type** |
|---|---|---|---|
| CEACAM1 | 634 | carcinoembryonic antigen-related cell adhesion molecule 1 (biliary glycoprotein) | other |
| IFIT3 | 3437 | interferon-induced protein with tetratricopeptide repeats 3 | other |
| IFITM3 | 10410 | interferon induced transmembrane protein 3 | other |
| OAS1 | 4938 | 2-5-oligoadenylate synthetase 140/46kDa | enzyme |
| VCAN | 1462 | versican | other |

Alternatively or additionally, the risk for developing T1D can be determined based on the up-regulation of one or more genes selected from erythrocyte-related genes listed in Table 3 as compared to a corresponding control level. In some embodiments, a predetermined control level may be used.

**Table 3. Erythrocyte-related genes whose up-regulation indicates risk for developing T1D**

| **Gene Symbol** | **Gene Bank ID** | **Gene Name** |
|---|---|---|
| CTTN | 2017 | cortactin |
| DCAF12 | 25853 | DDB1 and CUL4 associated factor 12 |
| EPB42 | 2038 | erythrocyte membrane protein band 4.2 |
| GMPR | 2766 | guanosine monophosphate reductase |
| MBNL3 | 55796 | muscleblind-like splicing regulator 3 |
| NFIX | 4784 | nuclear factor I/X (CCAAT-binding transcription factor) |
| NPRL3 | 8131 | nitrogen permease regulator-like 3 (*S. cere-visiae*) |
| SELENBP1 | 8991 | selenium binding protein 1 |
| SLC4A1 | 6521 | solute carrier family 4 (anion exchanger), member 1 (Diego blood group) |
| SLC6A8 | 6535 | solute carrier family 6 (neurotransmitter transporter), member 8 |
| TMEM158 | 25907 | transmembrane protein 158 |
| TNS1 | 7145 | tensin 1 |
| TRIM58 | 25893 | tripartite motif containing 58 |
| VWCE | 220001 | von Willebrand factor C and EGF domains |

In some further embodiments, the gene profile to be determined or measured comprises at least one gene selected from the group consisting of ADM, ALPL, ASGR2, BCL6, C19orf59, C3AR1, C4BPA, C5orf4, CACNG6, CBS, CCL8, CEACAM1, CLC, CNN2, CPPED1, CREB5, CTTN, CYSTM1, DCAF12, DEFA1 (includes others), DSC2, DYSF, EMR3, EPB42, EPB49, FAM129A, FAM46C, FOLR3, GMPR, GPR84, HBD, HLA-C, IFIT3, IFITM3, IGF2BP2, IRF5, KIAA1324, KRT1, LILRB3, LRG1, MAFB, MBNL3, MMP9, MX1, MYOF, NFIX, NPCDR1, NPRL3, OAS1, OLIG2, OSBP2, PHOSPHO1, PMP22, PROK2, PRSS33, RAB31, RBPMS2, RFX2, RNASE2, ROPN1L, RRP12, SEC14L1, SELENBP1, SIRPB1, SLC22A4, SLC29A1, SLC4A1, SLC6A8, SMOX, SNCA, SORT1, TGM3, TMEM158, TNS1, TRIM58, TSHZ3, VCAN, VNN1, VWCE, AHI1, C17orf97, C9orf123, CCL4L1/CCL4L2, CENPA, CEP55, COMMD6, COX7B, CYP4F3, DTL, EPSTI1, ERAP2, GBP5, GLT25D2, GNLY, HINT1, HIST1H4A (includes others), HLA-DPB1, HMMR, HOXB2, IFI27, IFI44, IL18RAP, ITGB1BP1, KLRD1, KLRF1, PRSS23, PTER, RPL26, RPS27L, RSL24D1, SAMD9L, SCOC, SETD9, SUB1, TMEM126B, XAF1, ANXA3, CASP5, HERC5, IFI44, IFI44L, IFIT2, OAS3, PI3, and RSAD2.

In some still further embodiments, said individual has an increased risk for developing T1D if in said gene profile at least one gene selected from the group consisting of ADM, ALPL, ASGR2, BCL6, C19orf59, C3AR1, C4BPA, C5orf4, CACNG6, CBS, CCL8, CEACAM1, CLC, CNN2, CPPED1, CREB5, CTTN, CYSTM1, DCAF12, DEFA1 (includes others), DSC2, DYSF, EMR3, EPB42, EPB49, FAM129A, FAM46C, FOLR3, GMPR, GPR84, HBD, HLA-C, IFIT3, IFITM3, IGF2BP2, IRF5, KIAA1324, KRT1, LILRB3, LRG1, MAFB, MBNL3, MMP9, MX1, MYOF, NFIX, NPCDR1, NPRL3, OAS1, OLIG2, OSBP2, PHOS-PHO1, PMP22, PROK2, PRSS33, RAB31, RBPMS2, RFX2, RNASE2, ROPN1L, RRP12, SEC14L1, SELENBP1, SIRPB1, SLC22A4, SLC29A1, SLC4A1, SLC6A8, SMOX, SNCA, SORT1, TGM3, TMEM158, TNS1, TRIM58, TSHZ3, VCAN, VNN1, and VWCE is up-regulated as compared to a control gene profile.

In some even further embodiments, said individual has an increased risk for developing T1D if in said gene profile at least one gene selected from the group consisting of AHI1, C17orf97, C9orf123, CCL4L1/CCL4L2, CENPA, CEP55, COMMD6, COX7B, CYP4F3, DTL, EPSTI1, ERAP2, GBP5, GLT25D2, GNLY, HINT1, HIST1H4A (includes others), HLA-DPB1, HMMR, HOXB2, IFI27, IFI44, IL18RAP, ITGB1BP1, KLRD1, KLRF1, PRSS23, PTER, RPL26, RPS27L, RSL24D1, SAMD9L, SCOC, SETD9, SUB1, TMEM126B, and XAF1 is down-regulated as compared to a control gene profile. In some preferred embodiments, said individual has an increased risk for developing T1D if KLFR1 (Killer cell lectin-like receptor subfamily F, member 1; Gene Bank ID: 51348) is down-regulated as compared to a control gene profile.

In some still further embodiments, said individual has an increased risk for developing T1D if in said gene profile at least one gene selected from the group consisting of ANXA3, CASP5, HERC5, IFI44, IFI44L, IFIT2, OAS3, PI3, and RSAD2 is updown-regulated as compared to control gene profile. As used herein, the term "updown-regulated" refers to three alternative situations: i) said gene is up-regulated in some subjects while down-regulated in other subjects; ii) some alternative probes for a given gene show up-regulation while some other probes for the same gene show down-regulation; and iii) before seroconversion, said gene shows both up-regulation and down-regulation de-pending on the measurement time point used.

Optionally, the present methods may further comprise determining variations in the subject's gene profile at different time points in order to monitor, prior to seroconversion, any changes in or the development of the risk for T1D.

A preferred control sample or control gene profile for use in the present methods is a case-matched sample or profile. Case-matching may be made, for instance, on the basis of one of more of the following criteria: age, date of birth, place of birth, gender, predisposition for T1D, HLA status and any relevant demographic parameter. In some embodiments, said control sample or profile consists of a pool of, preferably case-matched, relevant control samples or profiles. In some embodiments, said control prolife has been predetermined prior to predicting a risk of T1D in an individual in accordance with the present disclosure.

Optionally, before to be compared with the control gene profile, the expression level of genes, in particular one or more genes in any of the preferred expression profiles set forth above, are normalized using the expression level of an endogenous control gene having a stable expression in different samples, such as GAPDH.

Suitable samples for use in accordance with the present disclosure include, but are not limited to, tissue samples (e.g. pancreatic samples) and blood samples including whole blood, serum, plasma, peripheral blood mononuclear cells and any purified blood cell type. In essence, any biological sample which contains RNA, preferably mRNA or any other RNA species which represents the genes of interest, may be used for the present purposes. In some embodiments, the sample to be analyzed is extracted total whole-blood RNA or, if desired, the sample may consist of isolated mRNA or any other RNA species representing the genes of interest.

In some embodiments, the subject is a human subject, preferably a child or an adolescent. In some more preferred embodiments, said subject does not show any signs of seroconversion. As used herein, the terms "subject" and "individual" are interchangeable.

The expression level of a gene may be determined by a variety of techniques. In particular, the expression level of a gene may be determined by measuring the quantity of RNA, preferably mRNA or any other RNA species representing the gene of interest, using methods well known in the art. Non-limiting examples of suitable methods include real time (RT) quantitative or semi-quantitative PCR. Primers suitable for these methods may be easily designed by a skilled person.

Other non-limiting ways of measuring the quantity of RNA, preferably mRNA or any other RNA species representing the gene of interest, include transcriptome approaches, in particular DNA microarrays. Generally, when it is the quantity of mRNA that is to be determined, test and control mRNA samples are reverse transcribed and labelled to generate cDNA probes. The probes are then hybridized to an array of complementary nucleic acids immobilized on a solid support. The array is configured such that the sequence and position of each member of the array is known. Hybridization of a labelled probe with a particular array member indicates that the sample from which the probe was derived expresses that gene. Non-limiting examples of commercially available microarray systems include Affymetrix GeneChip™ and Illumina BeadChip. Next Generation Sequencing (NGS) methods may also be used.

If desired, the quantity of RNA, preferably mRNA any other RNA species representing the gene of interest, may also be determined or measured by conventional hybridization-based assays such as Northern blot analysis.

In another aspect, the present disclosure relates to an *in vitro* kit for predicting, before seroconversion, a risk of a subject T1D. The kit may be used in any one of the methods of the present disclosure. Typically, the kit may comprise a pair of primers and/or a probe specific to one or more genes in any of the preferred expression profiles set forth above, preferably in the expression profile of Table 1 and/or Table 2. A skilled person can easily design suitable primers and/or probes taking into account specific requirements of a technique to be applied.

The kit may further comprise means for detecting the hybridization of the probes with nucleotide molecules, such as mRNA or cDNA, representing genes expressed in a test sample and/or means for amplifying and/or detecting the nucleotide molecules representing the genes expressed in the test sample by using the pairs of primers.

Other optional components in the kit include a compartmentalized carrier means, one or more buffers (e.g. block buffer, wash buffer, substrate buffer, etc.), other reagents, positive or negative control samples, etc.

The kit may also comprise a computer readable medium comprising computer-executable instructions for performing any method of the present disclosure.

### EXAMPLES

### Example 1: Transcriptional signatures in children genetically at-risk of T1D

### METHODS

### Sample collection

All children studied were participants in the Finnish Type 1 Diabetes Prediction and Prevention Project (DIPP). Subjects identified as at risk for T1D based on their HLA haplotype were followed prospectively from birth. The protocol of the DIPP study has been approved by the Ethical Committees of the University Hospitals of Turku, Oulu and Tampere, and a written consent according to the Declaration of Helsinki was obtained from all study subjects and/or their parents. At the time of sample collection, 2.5 ml of venous blood was drawn into PAXgene Blood RNA tubes (PreAnalytix) at the DIPP study clinic, in Turku, Finland. The samples were incubated for 2 hours at room temperature and then stored at -70 °C until analyzed. Islet cell autoantibodies (ICA), insulin autoantibodies (IAA) and autoantibodies to glutamic acid decarboxylase 65 (GAD-65), islet antigen-2 (IA-2), and zinc transporter 8 (ZnT8) were measured from all individuals. Of 28 subjects in this study, nine were sampled starting before or at the time of the appearance of autoantibodies (seroconversion) (S1-5 and S7-10, Table 4); cases S3, S6, S7, and S10 progressed to T1D and the other six did not. The remaining 18 children (D1-18, Table 4) were all sampled starting after seroconversion and all progressed to T1D (D1-18). Figure 1 illustrates the timing of the blood draws for all case children. A persistently autoantibody negative control child was matched with each case, based on the date and place of birth, gender and *HLA-DQB1* genotype (data not shown). In all, 368 blood samples (191 from seroconverters and TID children, and 177 from healthy controls) were processed for genome-wide transcriptional analysis.

### RNA isolation

Total whole-blood RNA was extracted from the samples using PAXgene Blood RNA kit (Qiagen) according to manufacturer's instructions. RNA quality and quantity was determined using NanoDrop ND-1000 (Thermo Scientific) and Experion Automated Electrophoresis System (Bio-Rad Laboratories).

### Affymetrix Human Genome U219 array hybridizations

For all samples, 50 ng of total RNA was processed to cDNA with Ovation RNA amplification system v2, including the Ovation whole blood reagent (NuGEN Technologies). The amplified cDNA was subsequently biotin-labelled and fragmented with Encore biotin module (NuGEN Technologies). Samples were hybridized to GeneChip Human Genome U219 array plate with specific protocols for using the GeneTitan Hybridization, Wash and Stain Kit for 3' IVT Array Plates (Affymetrix). GeneTitan MC Instrument was used to hybridize, wash, stain and scan the arrays. Affymetrix GeneChip Command Consol 3.1 was used to control GeneTitan hybridization process and in summarizing probe cell intensity data (CEL file generation).

### Illumina Human-WG6 v2 array hybridizations

For seroconverted case-control pairs 1-6, 500 ng of total RNA was amplified and reverse transcribed with RiboAmp OA 1 round RNA amplification kit (Applied Biosystems/Arcturus). cRNA was biotinylated during the overnight (14 h) IVT reaction with Illumina total prep RNA amplification kit (Applied Biosystems/Ambion). cRNA was purified with RiboAmp OA 1 round RNA amplification kit. For seroconverted case-control pairs 7-10, 100 ng of total RNA was amplified and reverse transcribed with Ovation RNA amplification system v2, including the Ovation whole blood reagent (NuGEN Technologies). 5 µg of cDNA was labelled according to NuGEN's Illumina solution protocol, application note #2. cDNA concentration and quality was controlled with Experion automated electrophoresis system.

1.5 µg of each sample was hybridized to Sentrix human WG6 v2 expression bead chips (Illumina) at 58 °C overnight (18 h) according to Illumina whole-genome gene expression direct hybridization protocol, revision A. Hybridizations were detected with 1 µg ml⁻¹ Cyanine3-streptavidine (General Electric Healthcare Life Sciences). Chips were scanned with Illumina bead array reader. The expression intensities were extracted with Bead Studio version 2.3.41 or 3.3 without any normalization or background subtraction.

### Illumina Human HT-12 v3 array hybridizations

For all T1D progressor case-control pairs, 100 ng of total RNA was amplified to cDNA with Ovation RNA amplification system v2, including the Ovation whole blood reagent (NuGEN Technologies). The amplified cDNA was labelled according to NuGEN's Illumina solution protocol, application note #2. 750 ng of each cDNA was hybridized to Human HT-12 Expression BeadChips, version 3 (Illumina) at 58 °C overnight (18 h) according to Illumina whole-genome gene expression direct hybridization protocol, revision A. Hybridizations were detected with 1 µg ml⁻¹ Cyanine3-streptavidine (General Electric Healthcare Life Sciences). Chips were scanned with Illumina bead array reader, BeadScan software version 3.5. The expression intensities were extracted with GenomeStudio2008 v1 without any normalization or background subtraction.

### Microarray data processing and statistical analysis

The gene expression microarray data were preprocessed using the RMA procedure for the Affymetrix data and quantile normalization for the Illumina data using R/Bioconductor. Differentially expressed genes were identified by comparing subjects who had seroconverted to autoantibody positivity or progressed to clinical T1D with their matched healthy controls. Since the follow-up series are not fully synchronized in time between the different individuals, we first applied a similar approach as in Elo, *et al* 2010 and Huang, *et al* 2001 that avoids the need of a direct alignment. More specifically, for each individual, the expression intensity value of a particular probe/probeset *x* at each time point was given a *z*-score, defined as *z*=*(x-m)s*⁻¹*,* where *m* is the mean and s is the standard deviation calculated using all the time points in the matched control time series. Such *z*-scores penalize those probes/probesets that have high variability in the control series. To identify significant up- or down-regulation across the individuals, the rank product algorithm was applied to the case-wise maximum/minimum z-scores . Genes with false discovery rate (FDR) below 0.05, estimated as the percentage of false positive predictions (pfp), were considered as differentially regulated. To focus on T1D- or seroconversion-specific changes, the same procedure was applied to the data after swapping the case and control pairs. Only those probes were retained that did not pass the criterion FDR <0.05 in the swapped analysis. Additionally, we required that the genes were determined as present in more than 50% of all individuals (at least in one sample per individual) using a two-component Gaussian Mixture model for the Affymetrix data and detection P values for the Illumina data, as recommended by Painter, *et al* 2011. Finally, the findings from Affymetrix and Illumina arrays were compared, and genes showing concordant changes were regarded as final, validated findings (data not shown).

### Pathway analysis

The functional annotation tool DAVID (Database for Annotation, Visualization and Integrated Discovery) was used to identify enriched biological pathways (BBID, BioCarta, KEGG, Panther, Reactome) among the regulated genes. Pathways with FDR below 20% were reported (modified Fisher's exact test).

### Differentially expressed genes with functions related to the innate immunity responses

The human genes with literature-annotated function in the innate immunity were downloaded from www.innatedb.com. The enrichment of these genes among our differentially expressed genes was calculated using Fisher's exact test.

### Transcription binding motif analysis

Overrepresented transcription factor binding sites among the regulated genes were identified using the transcription factor target sets in the Molecular Signatures Database (MSigDB). Each target set in the database contains those genes that share a transcription factor binding site defined in the TRANSFAC (version 7.4) database and is annotated by a TRANSFAC record. Binding sites with P value below 0.05 were reported (hypergeometric test).

### Transcription module analysis

A module based method modified from Chaussabel, *et al* 2008 was used to survey coordinately expressed sets of genes (modules) functionally annotated with literature search. The gene symbols belonging to each module were downloaded from www.biir.net/modules, and compared to the differentially expressed genes. The genes not belonging to any of the modules presented in Chaussabel, *et al* 2008 were excluded from the analysis. The significance of overlap of each module with each list of differentially expressed genes was calculated using Fisher's exact test. Modules up- or down-regulated with the Benjamini-Hochberg corrected P value below 0.05 were visualized in the module maps with red or blue (up- and down-regulation, respectively), the intensity of the color corresponding to the percentage of the regulated genes in the module.

### Data analysis by time-windows

In order to identify transcripts active in different phases of the autoimmune process, the rank product method (R/Bioconductor RankProd package) was applied to find differentially expressed genes between the cases and their controls in five time-windows: before seroconversion, at seroconversion, 6-18 months after seroconversion, 1-2 years before clinical T1D diagnosis, and close to clinical T1D (Figure 1). The division was based on the maximal sample representation in each time-window. Inside each time-window, the fold change between the case and the matched control was calculated using linear inter/extrapolation, which takes into account the longitudinal profile of each gene. More specifically, the control sample series were matched to the time points of the case sample series. For the time points which were inside the range of real control time points this was performed by linear interpolation. For the time points needed outside this range, the expression values were approximated by constant extrapolation (set equal to the closest real measurement). Since independence of the replicates is assumed by the rank product method, only one sample per replicate was included in the analysis. Therefore, if several samples from a child were available inside the time-window, the maximum or minimum value was chosen for the detection of up- or down-regulated genes, respectively. Genes with false discovery rate (FDR) below 0.05 were considered differentially regulated. Additionally, we required that the genes were determined as present in more than 50% of all individuals (at least in one sample per individual) using a two-component Gaussian Mixture model for the Affymetrix data and detection P values for the Illumina data, as recommended by Painter, *et al* 2011. Finally, the findings from Affymetrix and Illumina arrays were compared, and genes showing concordant changes were regarded as final, validated findings (data not shown).

In addition to comparing seroconverted children with their controls, we did an additional comparison between those seroconverted children (cases S3, S7 and S10) who had later progressed to T1D, and those who have not been diagnosed with T1D to-date. An unpaired two group rank product analysis was performed to compare the affected and the unaffected cases. The analysis was performed for three time-windows: before seroconversion, at seroconversion, and 6-18 months after seroconversion.

Affymetrix and Illumina data were combined using the gene symbols provided by IPA. To combine the data from the Illumina WG-6 v2 and HT-12 arrays, the probes between the array types were matched based on their sequence similarity. 70% of the probes in Human WG-6 v2 array had remained completely identical to the corresponding probes in the Human HT-12 arrays. In addition, we mapped 6% of the probes using the following criteria: At least 24 consecutive bases (out of 50) were required to be totally identical between the probes. One different base was allowed in the middle if the sequences were otherwise identical at the length of 25 consecutive bases. The probes also had to target the same gene according to annotation. The remaining 24% of the probes were excluded from the analyses, for which the WG-6 v2 and HT-12 data needed to be combined.

### Immunochip data processing and statistical analysis

Genomic DNA was extracted from whole blood using the salting-out method in the Immunogenetics laboratory, University of Turku (Finland). Thereafter, samples were processed at the Department of Genetics, University Medical Centre Groningen (The Netherlands). The DNA quality and concentration was measured by fluorescence in the Synergy HT Multi-Mode Microplate Reader (Biotek) using the PicoGreen® dsDNA quantitation assay (Invitrogene) according to the manufacturer's instructions. Samples were diluted appropriately to a final concentration of 50 ng/µl and measured with NanoDrop 2000c (Thermo Scientific) prior to hybridization 200 ng of genomic DNA was amplified, enzymatically fragmented and hybridized onto the HumanImmuno BeadChips (Illumina) at 48°C overnight (20 h) according to the Illumina's Infinium HD Ultra Assay protocol. The staining of the arrays was performed using the Freedom EVO 100 robot (Tecan) and scanned with the iScan Reader (Illumina). Bead intensity data was analysed using the Genotyping Module version 1.9.4 of Genome Studio version 2011.1 (Illumina). NCBI build 36 (hg18) mapping was used (Illumina manifest file Immuno_BeadChip_11419691_B.bpm) for SNP mapping. A cluster set based on 196,524 autosomal and X-chromosome variants was applied to all samples. Quality control of the data and linkage disequilibrium (LD) pruning was performed using PLINK v1.07. First, non-polymorphic markers (N-17216) and markers with duplicated rs identifiers (N-864) were removed. Next, samples with a call rate below 95%, as well as SNPs with a call rate below 98% were excluded. Markers were excluded when they deviated from Hardy-Weinberg equilibrium in controls (P<0.0001). Due to our sample size we decided to focus on common markers only, thus SNPs with minor allele frequency below 10% were removed (N=80,259). Finally we pruned our dataset based on LD between markers if their r2 was greater than 0.8. Our final dataset comprised 30,463 SNPs. The effects of SNPs on gene expression were surveyed within 250 kb to both directions from each differentially expressed gene, using Affymetrix gene coordinates. A linear model was fit for genotype ∼ gene expression and a p-value was calculated for the null hypothesis that genotype has no effect on gene expression (slope = 0). A Benjamini-Hochberg correction was applied for the p-values and effects with FDR < 0.05 were considered significant (data not shown). These *cis* eQTL effects were then validated in a larger study with 5311 human whole-blood samples by Westra et al. (Nature Genetics 2013). The SNPs that had an eQTL effect on our differentially expressed genes were searched for associations with autoimmune diseases in GWA studies, as listed in Ricaño-Ponce and Wijmenga, Annual Review of Genomics and Human Genetics 2013 and/or T1Dbase (Burren, *et al* 2011). The proxies (r² > 0.8) for these SNPs were found based on HapMap3 (release 2) and 1000 Genomes in the CEU population panel by using SNP annotation and proxy search (http://www.broadinstitute.org/mpg/snap/Idsearch.php).

### Accession codes

The gene expression data discussed in this publication are accessible through GEO SuperSeries accession number GSE30211 (http://www.ncbi.nlm.nih.gov/geo/query/acc.cgi?acc=GSE30211).

### RESULTS

### Gene expression signatures detected during seroconversion to autoantibody positivity

When the transcriptional profiles of S1-10 (Table 1) were compared to their matched healthy controls, 109 unique genes were differentially expressed (false discovery rate, FDR<0.05), with 66% of genes being up-regulated and 34% being down-regulated. Genes residing in the genomic TID susceptibility loci (T1Dbase) included the up-regulated oncostatin M (*OSM*) and the down-regulated killer cell lectin-like receptor subfamily B member 1 (*KLRB1*) (Fig. 5). The up-regulated genes were enriched in the retinoic acid inducible gene 1 (RIG-I)-like receptor signaling pathway involved in recognizing viral dsRNA (data not shown).

Altogether, 12 unique transcription factor genes were identified as being differentially regulated in the seroconverted cases. Analysis of known gene and protein interactions among these transcription factors and other genes in the dataset using the Ingenuity Pathway Analysis (IPA) network neighborhood tool showed that interconnected interferon regulatory factors 5 and 7 (*IRF5* and *IRF7*) had several co-regulated network partners (Fig. 2). Both *IRF5* and *IRF7* are highly expressed in lymphoid tissues and drive the activation of a wide range of antiviral genes. Target genes in the identified network included for example the DEAD box protein 58 (*DDX58,* better known as Retinoic acid inducible gene 1, *RIG-I*), which codes a helicase involved in viral double stranded RNA recognition, as well as T1D-associated 2'-5'-oligoadenylate synthetase 1 (*OAS1*), which codes for an enzyme involved in activating RNAse L, resulting in viral RNA degradation.

### Gene expression during the autoantibody-positive period preceding clinical T1D

A comparison of the samples obtained from the 18 T1D progressors (D1-18, Table 4) to their matched controls revealed 472 differentially expressed genes (FDR<0.05; data not shown). Notably, gene expression was suppressed, as 73% of the differentially regulated genes in the T1D individuals were down-regulated. The up-regulated genes residing in the genomic TID susceptibility loci (T1Dbase) included the TRAF-type zinc finger domain containing 1 (*TRAFD1*) and the signal transducer and activator of transcription 2 (*STAT2*). In contrast, the T1D-associated grancalcin (*GCA*) and the von Hippel-Lindau binding protein 1 (*VBP1*) were down-regulated (Fig. 5). The top down-regulated pathways were related to immune system signaling, blood coagulation and complement (data not shown). Dopamine receptor mediated signalling pathway was the only enriched pathway among the up-regulated genes.

We identified upregulation of transcription factors promyelocytic leukemia (*PML*) and nuclear receptor coactivator 7 (*NCOA7*), as well as down-regulation of myoneurin (*MYNN*) both in the seroconverted individuals and in the T1D progressors. Of these, the best studied factor is the PML, which is the major structural component of nuclear PML bodies involved in various cellular activities including protein degradation, DNA damage and apoptosis. PML expression is induced by type I and II interferons and it is known to inhibit viral replication both in the nucleus and cytoplasm. Subsequently, numerous DNA and RNA viruses use PML nuclear body dissociation as their survival mechanism. In addition, PML acts as a cofactor for the promyelocytic leukemia zinc finger (PLZF, also known as ZBTB16) transcription factor in order to induce a specific set of interferon stimulated genes, such as *OAS1, STAT1,* and radical S-adenosyl methionine domain containing 2 (*RSAD2*) upon interferon stimulus. Interestingly, also the binding sites of many interferon regulated transcription factors were enriched in the promoters of the differentially expressed genes (data not shown). For instance, IRF7 and IRF8 binding sites were enriched among the up-regulated genes in both the seroconverted cases (P=0.000309 and 0.00173) and the T1D progressors (P=0.0184 and 0.00106). The up-regulated genes identified from the seroconverted cases were also enriched for IRF1 (P=0.0000329) and IRF2 (P=0.0268) motifs, as well as for the interferon stimulated response element (ISRE, P=0.000274), which is bound and regulated by interferon-stimulated gene factor (ISGF3), a heterotrimeric transcription factor complex consisting of IRF9, STAT1 and STAT2 protein subunits.

**Table 4. Summary of the seroconverted children (S) and T1D progressors (D) studied with genome-wide transcriptomics**

| **Case** | **Number of longitudinal RNA samples** | **First sample from seroconversion (months)** | **Last sample from seroconversion (months)** | **Time of T1D diagnosis** | **Age at T1D diagnosis (years)** |
|---|---|---|---|---|---|
| S1 | 8 | 0 | 23 | na | |
| S2 | 7 | -10 | 19 | na | |
| S3 | 7 | -12 | 12 | 20 months after last sample | 7.7 |
| S4 | 5 | 0 | 15 | na | |
| S5 | 4 | 0 | 14 | na | |
| S6 | 6 | 68 | 89 | 0 months after last sample | 8.6 |
| S7 | 9 | -37 | 12 | 22 months after last sample | 9.3 |
| S8 | 6 | -12 | 15 | na | |
| S9 | 10 | -40 | 3 | na | |
| S10 | 4 | -12 | 4 | 1 month after last sample | 2.4 |
| | | | | | |

| **Case** | **Number of longitudinal RNA samples** | **First sample from seroconversion (months)** | **First sample from diagnosis (months)** | **Time of T1D diagnosis** | **Age at T1D diagnosis (years)** |
|---|---|---|---|---|---|
| D1 | 7 | 17 | -33 | at last sample | 6.6 |
| D2 | 6 | 51 | -13 | 0.6 months after last sample | 6.6 |
| D3 | 12 | 59 | -9 | 20.5 months prior to last sample | 6.9 |
| D4 | 7 | 49 | -20 | at last sample | 7.8 |
| D5 | 8 | 39 | -31 | at last sample | 8.3 |
| D6 | 6 | 3 | -30 | 3.2 months after last sample | 8.8 |
| D7 | 4 | 76 | -10 | 1.4 months after last sample | 9.2 |
| D8 | 12 | 86 | -46 | 3.2 months after last sample | 12.1 |
| D9 | 7 | 85 | -24 | at last sample | 10 |
| D10 | 6 | 81 | -23 | 1.4 months after last sample | 12.5 |
| D11 | 8 | na | -32 | at last sample | 13 |
| D12 | 6 | 3 | -15 | at last sample | 2.3 |
| D13 | 5 | 3 | -16 | at last sample | 3 |
| D14 | 5 | 11 | -14 | at last sample | 3.2 |
| D15 | 8 | 3 | -27 | at last sample | 3.6 |
| D16 | 9 | 2 | -27 | 0.3 months after last sample | 3.8 |
| D17 | 4 | 3 | -12 | 2.9 months after last sample | 4.2 |
| D18 | 5 | 30 | -24 | 0.4 months after last sample | 4.9 |

### Identification of transcriptional signatures characteristic for distinct phases of the T1D autoimmune process

To identify transcripts that are active in different phases of the T1D autoimmune process, we divided and analyzed the data separately within five time-windows (Fig. 1). In the time-windows before, at, or 6-18 months after seroconversion, 124, 33, and 551 genes were identified as differentially expressed (FDR<0.05), respectively; only 14 genes were identified that were common to all three time windows (Fig. 6 and data not shown). During the intervals of 1-2 years before clinical diagnosis and at clinical diagnosis, 388 and 211 genes were differentially expressed between the cases and controls (Fig. 6 and Table S4). Different time windows showed enrichment of genes bearing different transcription factor binding sites. IRF and/or STAT binding sites were enriched in genes up-regulated before seroconversion, and in the interval between seroconversion and diagnosis (data not shown).

A modular data mining strategy that identifies coordinately expressed transcripts, often functionally related, was recently utilized to identify disease-specific transcriptional fingerprints in whole-blood analysis identifying the over-represented interferon module in systemic lupus erythematosus (SLE) and tuberculosis. By utilizing these predefined and literature-annotated modules, we observed that the changes in transcriptional signatures were dynamic relative to different stages of T1D pathogenesis (Fig. 3 and data not shown). However, a module consisting of interferon-induced transcripts (M3.1) was constantly showing changes at almost every stage of T1D development.

### Transcriptional signatures distinct for T1D progressors among the seroconverted individuals

Finally, we performed a separate time-window analysis for the seroconverted cases who later progressed to clinical T1D (cases S3, S6, S7 and S10, Table 4) compared to seroconverted cases who have remained non-diabetic for at least 68 months. For cases S3, S7, and S10, data before, at, and 6-18 months after seroconversion were available.

Before seroconversion, nine genes were identified as differentially regulated (FDR <0.05; data no shown). At seroconversion and 6-18 months after seroconversion the difference had increased to 13 and 54 genes, respectively. One of the genes showing constant and high upregulation in the progressors was Ribonuclease, RNase A family, 2 (*RNASE2,* also known as eosinophil-derived neurotoxin, *EDN,* Fig. 4). It encodes a secreted protein with several immunomodulatory functions. The ribonucleolytic activity of RNASE2 plays an important role in eosinophil-mediated antiviral activity against single-stranded RNA viruses. RNASE2 is an endogenous TLR2 ligand that could play a role in the induction of interferons. Most interestingly, *RNASE2* expression has been reported to be up-regulated in the PBMCs of patients with autoimmune diseases, such as rheumatoid arthritis (RA) and SLE.

### Identification of the genomic variants affecting the expression levels

In order to study the effect of possible single nucleotide polymorphisms (SNP) as the cause for the detected expression differences, we utilized a custom SNP array (Immunochip, Illumina) for genotyping. Using representative (LD pruned) SNP markers residing inside a window of +-250 kb around the gene coordinates, 118 differentially expressed genes had a *cis* eQTL (*cis* acting expressed quantitative trait loci) effect with 1-54 SNPs per gene (FDR <0.05, data not shown). Comparison to eQTLs recently identified in whole-blood from 5311 individuals validated 27% of the detected *cis* effects (Table S7). 14 of the identified eQTL genes (the identified SNPs or their proxies r²>0.8), such as Histone cluster 1 H2bd (*HIST1H2BD*)*,* have also been associated with autoimmune diseases in GWAS, as listed in Ricaño-Ponce and Wijmenga, Annual Review of Genomics and Human Genetics 2013and/or T1Dbase (data not shown). Interestingly, eQTL effects were found with SNPs adjacent to *IRF5, OAS1, OAS2, DDX58,* Transporter 2 ATP-binding cassette sub-family B (MDR/TAP) (*TAP2*), Indoleamine 2,3-dioxygenase 1 (*IDO1*) and Leukocyte immunoglobulin-like receptor subfamily A (with TM domain) member 5 (*LILRA5*), which were identified as the core of the central interferon stimulatory network in the seroconverted individuals (Fig. 2). Previously, SNPs overlapping the *TAP2* gene residing in the human MHC locus have been associated with T1D in several studies and the *IRF5* polymorphisms have been connected to SLE, RA, and IBD.

### DISCUSSION

This study provides a comprehensive analysis of transcriptional changes that occur over time in children at risk for or developing T1D, and provide a unique resource for new hypotheses explaining T1D biology. Our results demonstrate that T1D-specific changes are evident in the whole-blood transcriptome, affecting hundreds of genes. As the gene expression analysis was performed on whole-blood samples, the contribution of each blood cell subtype remains unclear. However, the module-based tool may suggest which cell types are important in different phases of T1D pathogenesis (Fig. 3 and data not shown). For example, neutrophil module was first activated, after which the neutrophil-associated transcripts were suppressed before diagnosis, consistent with a recent report on reduced numbers of circulating neutrophils during autoantibody positive phase as well as at onset of T1D in humans. Also the erythrocyte related module was activated at early time-windows and before diagnosis. Platelet module was activated after seroconversion until clinical T1D, which is in line with the reports on platelet hyperreactivity in T1D. Unexpectedly, the cytotoxic cell module was highly activated at the time of diagnosis, but not before.

Taken together, our results indicate the activation of type 1 interferon mediated innate immune system responses throughout the course of T1D development, and importantly even prior to seroconversion. The induction is temporal, as expected for this systemic response, but some individuals show several signature peaks during the follow-up period (Fig. 5). Interferons α and β were not differentially regulated in our data, indicating that the blood cells are showing an indirect response to cytokines produced elsewhere. For instance, enteroviral infections have been detected in the islets of T1D patients and in the beta cells of newly diagnosed patients. Such infections have also been detected a few months prior to seroconversion to autoantibody positivity and during the 6-month period preceding seroconversion. In agreement with our results, Reynier et al. (Reynier, *et al* 2010) recently reported that 12 interferon response genes are up-regulated in whole-blood samples of 30% of autoantibody-positive prediabetic children, but not in healthy controls or recently diagnosed T1D patients. Our data indicate that the interferon response can be detected even earlier, before the appearance of autoantibodies (Fig. 3), and that it consists of much larger number of genes than the previous report. In fact, comparison of the differentially regulated genes to the genes listed to be involved in functions related to human innate immune responses in the InnateDB provides us with 17.4% overlap for the seroconverted cases (19 genes, P=0.0000003) and 9.5% overlap with the T1D progressors (45 genes, P=value 0.0000013), respectively (data not shown). As one of the examples, the interferon-regulated IRF7 was identified as one of the central upstream regulators of gene expression in the seroconverted individuals. Recently, Heinig et al. (Heinig, *et al* 2010) performed an (eQTL) analysis in rat tissues and human monocytes, and identified IRF7-driven transcriptional network associating with T1D in previous genome wide association studies. As much as 67% (8/12) of the direct IRF7 target genes differentially expressed in our study (Fig. 2) are also present in the human IRF7 network identified by Heinig et al. Thus, these complementary approaches further highlight the role of the interferon pathway in T1D pathogenesis.

In addition to viral antigens, bacterial DNA, LPS, and flagellin are efficient triggers of TLR signaling and the interferon response, providing an intriguing link between the gut immune system and autoimmunity. Both preclinical and established T1D patients exhibit increased gut permeability, leading to enhanced immune responses in the intestinal tissues. Also, a preliminary study has shown that gut microbiome diversity is reduced in children progressing towards T1D compared to healthy controls. Moreover, the interferon response can be activated without infection by bacteria or viruses; for example, in SLE, IFNα production is driven by TLR7-mediated signaling and is induced by autoantibody-protein-RNA complexes derived from apoptotic cells. Also, endogenous TLR ligands such as RNASE2 that was identified to be up-regulated in the progressors could play a role in the induction of interferons. It has been shown that spontaneous interferon production drives autoimmune diabetes in NOD mice. Although interferons prepare cells for efficient antiviral defense during infection, these cytokines have also wider effects on immune cells, such as promoting dendritic cell maturation and activation, B cell survival and Ig isotype switching, and suppression of T regulatory cells.

In this study, the matching of the cases and controls was based on the *HLA-OQB1* genotype. To take into account further susceptibility alleles and alterations affecting the transcriptional changes, genome-wide SNP detection was performed for combinatorial analysis of the genome and transcriptome. This revealed that approximately 10% of the genes differentially expressed between the cases and controls were affected in *cis* by the genetic variation. The affected variants and genes have previously been linked to several autoimmune diseases, highlighting the shared genetic basis for these disorders. For example in the case of SLE, where strong type 1 interferon signature is also observed, the associated variations in *IRF5* have been shown to control cytokine responses upon TLR ligation. This pathway could be genetically linked also to T1D, since only a small part of the genetic factors conferring susceptibility to T1D have been identified. Alternatively, common gene variants influencing gene expression levels, in combination with additional factors, may contribute to the development of the disease. The majority of the expression changes, however, appeared not to be affected by the genetic variation between the individuals suggesting that the majority of the observed differences in gene expression reflect additional levels of regulation, such as epigenetic modifications. Ancillary measurements, such as proteomics and metabolomics data, could be used in combination with transcriptomics results to identify a combination of candidate molecules that may aid in the prediction of progression toward overt T1D or monitoring the activity of the clinical disease. In addition, the correlation of gene expression profiles to markers of virus infections in susceptible children could make it possible to explore the mechanisms leading to the activation of the interferon system.

### Example 2: Additional transcriptional signatures in children genetically at-risk of T1D

Another cohort of seroconverted and T1D DIPP children were analyzed (unpublished). Samples were collected as in Example 1. Of 16 subjects in this study, all were sampled starting before the time of the appearance of autoantibodies (seroconversion). Out of these, three children progressed to T1D. A persistently autoantibody negative control child was matched with each case, based on the date and place of birth, gender and HLA-DQB1 genotype (data not shown). In all, 183 blood samples were processed for genome-wide transcriptional analysis with GeneChip Human Genome U219 array plates as in Example 1. Data was analyzed as in Example 1, focusing on the time-window before seroconversion.

### Example 3: Comparison to other available data

The publicly available dataset of Ferreira et al. was preprocessed and analyzed for differential expression similarly as in Example 1. A total of 19,310 transcripts with a gene symbol in Ingenuity Knowledge base were identified from the dataset after removing multiple transcripts mapping to the same gene by selecting the representative transcript with largest IQR across all samples.

Each case child with samples before seroconversion (n=20) was matched with a healthy control child with the same gender and time of birth. For each gene, the maximum and minimum signal log ratio (SLR) between case samples before seroconversion and controls was calculated using linear inter/extrapolation. Differentially expressed genes were identified using rank product test corrected for multiple testing. Using a false discovery rate (FDR) cutoff <0.05 and SLR of 1 and -1 (at least 2-fold change resulted in 23 up-regulated and 1 down-regulated genes that overlapped with the results of Example 1 preceding seroconversion.

Microarray data preceding seroconversion (Example 1) were clustered hierarchically by Pearson correlation analysis independently for SLR data and expression intensity data. The number of clusters was estimated using elbow method. Each gene cluster was further analyzed using linear discriminant method to find the best scoring clusters that discriminates seroconverted cases from the controls. The gene lists of top scoring clusters were compared with the gene lists of Ferreira et al. 2014. Similarly, gene lists in the time-window before seroconversion in Example 2 were compared with the clusters.

Finally, the up-regulated genes common between all compared gene lists were selected and divided into three functionally different groups of interferon-regulated, non-interferon regulated, and erythrocyte-related gene sets shown in Tables 1, 2 and 3, respectively. Owing to their up-regulation in two or three datasets, the listed genes are particularly suitable for predicting, prior to seroconversion, the development of T1D in an individual.

It will be obvious to a person skilled in the art that, as the technology advances, the inventive concept can be implemented in various ways. The invention and its embodiments are not limited to the examples described above.

### REFERENCES

Burren OS, Adlem EC, Achuthan P, Christensen M, Coulson RM, Todd JA (2011). T1DBase: update 2011, organization and presentation of large-scale data sets for type 1 diabetes research. Nucleic Acids Res 39: D997-1001.
Chaussabel D, Quinn C, Shen J, Patel P, Glaser C, Baldwin N, Stichweh D, Blankenship D, Li L, Munagala I, Bennett L, Allantaz F, Mejias A, Ardura M, Kaizer E, Monnet L, Allman W, Randall H, Johnson D, Lanier A, Punaro M, Wittkowski KM, White P, Fay J, Klintmalm G, Ramilo O, Palucka AK, Banchereau J, Pascual V (2008). A modular analysis framework for blood genomics studies: application to systemic lupus erythematosus. Immunity 29: 150-164.
Elo LL, Mykkänen J, Nikula T, Järvenpää H, Simell S, Aittokallio T, Hyöty H, Ilonen J, Veijola R, Simell T, Knip M, Simell O, Lahesmaa R (2010). Early suppression of immune response pathways characterizes children with prediabetes in genome-wide gene expression profiling. J Autoimmun 35: 70-76.
Ferreira RC, Guo H, Coulson RM, Smyth DJ, Pekalski ML, Burren OS, Cutler AJ, Doecke JD, Flint S, McKinney EF, Lyons PA, Smith KG, Achenbach P, Beyerlein A, Dunger DB, Wicker LS, Todd JA, Bonifacio E, Wallace C, Ziegler AG (2014). A type I interferon transcriptional signature precedes autoimmunity in children genetically at-risk of type 1 diabetes. Diabetes. Online ahead of print Feb 21 2014.
Heinig M, Petretto E, Wallace C, Bottolo L, Rotival M, Lu H, Li Y, Sarwar R, Langley SR, Bauerfeind A, Hummel O, Lee YA, Paskas S, Rintisch C, Saar K, Cooper J, Buchan R, Gray EE, Cyster JG, Cardiogenics Consortium, Erdmann J, Hengstenberg C, Maouche S, Ouwehand WH, Rice CM, Samani NJ, Schunkert H, Goodall AH, Schulz H, Roider HG, Vingron M, Blankenberg S, Munzel T, Zeller T, Szymczak S, Ziegler A, Tiret L, Smyth DJ, Pravenec M, Aitman TJ, Cambien F, Clayton D, Todd JA, Hubner N, Cook SA (2010). A trans-acting locus regulates an anti-viral expression network and type 1 diabetes risk. Nature 467: 460-464.
Huang Q, Liu D, Majewski P, Schulte LC, Korn JM, Young RA, Lander ES, Hacohen N (2001). The plasticity of dendritic cell responses to pathogens and their components. Science 294: 870-875.
Painter MW, Davis S, Hardy RR, Mathis D, Benoist C, Immunological Genome Project Consortium (2011). Transcriptomes of the B and T lineages compared by multiplatform microarray profiling. J Immunol 186: 3047-3057.
Reynier F, Pachot A, Paye M, Xu Q, Turrel-Davin F, Petit F, Hot A, Auffray C, Bendelac N, Nicolino M, Mougin B, Thivolet C (2010). Specific gene expression signature associated with development of autoimmune type-I diabetes using whole-blood microarray analysis. Genes Immun 11: 269-278.
Ricaño-Ponce I and Wijmenga C (2013). Mapping of immune-mediated disease genes. Annu Rev Genomics Hum Genet 14:325-353.
Westra H-J, Peters MJ, Esko T, Yaghootkar H, Schurmann C, Kettunen J, Christiansen MW, Fairfax BP, Schramm K, Powell JE, Zhernakova A, Zhernakova DV, Veldink JH, Van den Berg LH, Karjalainen J, Withoff S, Uitterlinden AG, Hofman A, Rivadeneira F, 't Hoen PAC, Reinmaa E, Fischer K, Nelis M, Milani L, Melzer D, Ferrucci L, Singleton AB, Hernandez DG, Nalls MA, Homuth G, Nauck M, Radke D, Völker U, Perola M, Salomaa V, Brody J, Suchy-Dicey A, Gharib SA, Enquobahrie DA, Lumley T, Montgomery GW, Makino S, Prokisch H, Herder C, Roden M, Grallert H, Meitinger T, Strauch K, Li Y, Jansen RC, Visscher PM, Knight JC, Psaty BM, Ripatti S, Te-umer A, Frayling TM, Metspalu A, van Meurs JBJ, Franke L (2013). Systematic identification of trans-eQTLs as putative drivers of known disease associa-tions. Nat Genet 45: 1238-1243.

## Claims

1. A method of predicting, before seroconversion, a risk of a subject for Type 1 diabetes (T1D) comprising the following steps:
a) providing a sample obtained from said subject,
b) determining a gene expression profile of at least C17orf97 in said sample, and
c) using said gene expression profile for the prediction of the probability of the subject to develop T1D.

2. The method according to claim 1, wherein said method further comprises determining a gene expression profile of at least one further gene selected from the group consisting of ADM, ALPL, ASGR2, BCL6, C19orf59, C3AR1, C4BPA, C5orf4, CACNG6, CBS, CCL8, CEACAM1, CLC, CNN2, CPPED1, CREB5, CTTN, CYSTM1, DCAF12, DEFA1, DSC2, DYSF, EMR3, EPB42, EPB49, FAM129A, FAM46C, FOLR3, GMPR, GPR84, HBD, HLA-C, IFIT3, IFITM3, IGF2BP2, IRF5, KI-AA1324, KRT1, LILRB3, LRG1, MAFB, MBNL3, MMP9, MX1, MYOF, NFIX, NPCDR1, NPRL3, OAS1, OLIG2, OSBP2, PHOSPHO1, PMP22, PROK2, PRSS33, RAB31, RBPMS2, RFX2, RNASE2, ROPN1L, RRP12, SEC14L1, SELENBP1, SIRPB1, SLC22A4, SLC29A1, SLC4A1, SLC6A8, SMOX, SNCA, SORT1, TGM3, TMEM158, TNS1, TRIM58, TSHZ3, VCAN, VNN1, VWCE, AHI1, C9orf123, CCL4L1/CCL4L2, CENPA, CEP55, COMMD6, COX7B, CYP4F3, DTL, EP-STI1, ERAP2, GBP5, GLT25D2, GNLY, HINT1, HIST1H4A, HLA-DPB1, HMMR, HOXB2, IFI27, IFI44, IL18RAP, ITGB1BP1, KLRD1, KLRF1, PRSS23, PTER, RPL26, RPS27L, RSL24D1, SAMD9L, SCOC, SETD9, SUB1, TMEM126B, XAF1, ANXA3, CASP5, HERC5, IFI44, IFI44L, IFIT2, OAS3, PI3, and RSAD2.

3. The method according to any one of the preceding claims, wherein said subject has a HLA-conferred risk of T1D.

4. The method according to any one of the preceding claims, wherein said sample is a tissue sample, a whole blood sample, or a sample comprising peripheral mononuclear cells.

5. Use of a kit in a method according to any one of claims 1 to 4, wherein said kit comprises one or more testing agents capable of detecting the expression level of C17orf97 in a biological sample obtained from a subject whose risk for developing T1D is to be determined.

6. The use according to claim 5, wherein said kit further comprises one or more testing agents capable of detecting the expression level of one or more genes selected from the group consisting of ADM, ALPL, ASGR2, BCL6, C19orf59, C3AR1, C4BPA, C5orf4, CACNG6, CBS, CCL8, CEACAM1, CLC, CNN2, CPPED1, CREB5, CTTN, CYSTM1, DCAF12, DEFA1, DSC2, DYSF, EMR3, EPB42, EPB49, FAM129A, FAM46C, FOLR3, GMPR, GPR84, HBD, HLA-C, IFIT3, IFITM3, IGF2BP2, IRF5, KIAA1324, KRT1, LILRB3, LRG1, MAFB, MBNL3, MMP9, MX1, MYOF, NFIX, NPCDR1, NPRL3, OAS1, OLIG2, OSBP2, PHOSPHO1, PMP22, PROK2, PRSS33, RAB31, RBPMS2, RFX2, RNASE2, ROPN1L, RRP12, SEC14L1, SELENBP1, SIRPB1, SLC22A4, SLC29A1, SLC4A1, SLC6A8, SMOX, SNCA, SORT1, TGM3, TMEM158, TNS1, TRIM58, TSHZ3, VCAN, VNN1, VWCE, AHI1, C9orf123, CCL4L1/CCL4L2, CENPA, CEP55, COMMD6, COX7B, CYP4F3, DTL, EPSTI1, ERAP2, GBP5, GLT25D2, GNLY, HINT1, HIST1H4A, HLA-DPB1, HMMR, HOXB2, IFI27, IFI44, IL18RAP, ITGB1BP1, KLRD1, KLRF1, PRSS23, PTER, RPL26, RPS27L, RSL24D1, SAMD9L, SCOC, SETD9, SUB1, TMEM126B, XAF1, ANXA3, CASP5, HERC5, IFI44, IFI44L, IFIT2, OAS3, PI3, and RSAD2.

## Patentansprüche

1. Verfahren zum Vorhersagen eines Risikos eines Probanden für Typ-1-Diabetes (T1D) vor der Serokonversion, umfassend die folgenden Schritte:
a) Bereitstellen einer von dem Probanden gewonnenen Probe,
b) Bestimmen eines Genexpressionsprofils von mindestens C17orf97 in der Probe und
c) Verwenden des Genexpressionsprofils zur Vorhersage der Wahrscheinlichkeit des Probanden, T1D zu entwickeln.

2. Verfahren gemäß Anspruch 1, wobei das Verfahren ferner umfasst, ein Genexpressionsprofil von mindestens einem weiteren Gen zu bestimmen, ausgewählt aus der Gruppe, bestehend aus ADM, ALPL, ASGR2, BCL6, C19orf59, C3AR1, C4BPA, C5orf4, CACNG6, CBS, CCL8, CEACAM1, CLC, CNN2, CPPED1, CREB5, CTTN, CYSTM1, DCAF12, DEFA1, DSC2, DYSF, EMR3, EPB42, EPB49, FAM129A, FAM46C, FOLR3, GMPR, GPR84, HBD, HLA-C, IFIT3, IFITM3, IGF2BP2, IRF5, KIAA1324, KRT1, LILRB3, LRG1, MAFB, MBNL3, MMP9, MX1, MYOF, NFIX, NPCDR1, NPRL3, OAS1, OLIG2, OSBP2, PHOSPHO1, PMP22, PROK2, PRSS33, RAB31, RBPMS2, RFX2, RNASE2, ROPN1L, RRP12, SEC14L1, SELENBP1, SIRPB1, SLC22A4, SLC29A1, SLC4A1, SLC6A8, SMOX, SNCA, SORT1, TGM3, TMEM158, TNS1, TRIM58, TSHZ3, VCAN, VNN1, VWCE, AHI1, C9orf123, CCL4L1/CCL4L2, CENPA, CEP55, COMMD6, COX7B, CYP4F3, DTL, EP-STI1, ERAP2, GBP5, GLT25D2, GNLY, HINT1, HIST1H4A, HLA-DPB1, HMMR, HOXB2, IFI27, IFI44, IL18RAP, ITGB1BP1, KLRD1, KLRF1, PRSS23, PTER, RPL26, RPS27L, RSL24D1, SAMD9L, SCOC, SETD9, SUB1, TMEM126B, XAF1, ANXA3, CASP5, HERC5, IFI44, IFI44L, IFIT2, OAS3, PI3 und RSAD2.

3. Verfahren gemäß einem der vorhergehenden Ansprüche, wobei der Proband ein HLA-vermitteltes Risiko für T1D aufweist.

4. Verfahren gemäß einem der vorhergehenden Ansprüche, wobei es sich bei der Probe um eine Gewebeprobe, eine Ganzblutprobe oder eine periphere mononukleäre Zellen umfassende Probe handelt.

5. Verwendung eines Kits bei einem Verfahren gemäß einem der Ansprüche 1 bis 4, wobei der Kit ein oder mehrere Testmittel umfasst, die fähig sind, den Expressionsspiegel von C17orf97 in einer von einem Probanden, dessen Risiko für das Entwickeln von T1D bestimmt werden soll, gewonnenen biologischen Probe nachzuweisen.

6. Verwendung gemäß Anspruch 5, wobei der Kit ferne ein oder mehrere Testmittel umfasst, die fähig sind, den Expressionsspiegel von einem oder mehreren Genen nachzuweisen, ausgewählt aus der Gruppe bestehend aus ADM, ALPL, ASGR2, BCL6, C19orf59, C3AR1, C4BPA, C5orf4, CACNG6, CBS, CCL8, CEACAM1, CLC, CNN2, CPPED1, CREB5, CTTN, CYSTM1, DCAF12, DEFA1, DSC2, DYSF, EMR3, EPB42, EPB49, FAM129A, FAM46C, FOLR3, GMPR, GPR84, HBD, HLA-C, IFIT3, IFITM3, IGF2BP2, IRF5, KIAA1324, KRT1, LILRB3, LRG1, MAFB, MBNL3, MMP9, MX1, MYOF, NFIX, NPCDR1, NPRL3, OAS1, OLIG2, OSBP2, PHOSPHO1, PMP22, PROK2, PRSS33, RAB31, RBPMS2, RFX2, RNASE2, ROPN1L, RRP12, SEC14L1, SELENBP1, SIRPB1, SLC22A4, SLC29A1, SLC4A1, SLC6A8, SMOX, SNCA, SORT1, TGM3, TMEM158, TNS1, TRIM58, TSHZ3, VCAN, VNN1, VWCE, AHI1, C9orf123, CCL4L1/CCL4L2, CENPA, CEP55, COMMD6, COX7B, CYP4F3, DTL, EP-STI1, ERAP2, GBP5, GLT25D2, GNLY, HINT1, HIST1H4A, HLA-DPB1, HMMR, HOXB2, IFI27, IFI44, IL18RAP, ITGB1BP1, KLRD1, KLRF1, PRSS23, PTER, RPL26, RPS27L, RSL24D1, SAMD9L, SCOC, SETD9, SUB1, TMEM126B, XAF1, ANXA3, CASP5, HERC5, IFI44, IFI44L, IFIT2, OAS3, PI3 und RSAD2.

## Revendications

1. Procédé de prédiction, avant séroconversion, du risque qu'un sujet présente un diabète de type 1 (T1D), comprenant les étapes suivantes :
a) fourniture d'un échantillon obtenu chez ledit sujet,
b) détermination d'un profil d'expression génique d'au moins C17orf97 dans ledit échantillon, et
c) utilisation dudit profil d'expression génique pour la prédiction de la probabilité que le sujet développe un T1D.

2. Procédé selon la revendication 1, ledit procédé comprenant en outre la détection d'un profil d'expression génique d'au moins un autre gène choisi dans le groupe constitué de ADM, ALPL, ASGR2, BCL6, C19orf59, C3AR1, C4BPA, C5orf4, CACNG6, CBS, CCL8, CEACAM1, CLC, CNN2, CPPED1, CREB5, CTTN, CYSTM1, DCAF12, DEFA1, DSC2, DYSF, EMR3, EPB42, EPB49, FAM129A, FAM46C, FOLR3, GMPR, GPR84, HBD, HLA-C, IFIT3, IFITM3, IGF2BP2, IRF5, KIAA1324, KRT1, LILRB3, LRG1, MAFB, MBNL3, MMP9, MX1, MYOF, NFIX, NPCDR1, NPRL3, OAS1, OLIG2, OSBP2, PHOSPHO1, PMP22, PROK2, PRSS33, RAB31, RBPMS2, RFX2, RNASE2, ROPN1L, RRP12, SEC14L1, SELENBP1, SIRPB1, SLC22A4, SLC29A1, SLC4A1, SLC6A8, SMOX, SNCA, SORT1, TGM3, TMEM158, TNS1, TRIM58, TSHZ3, VCAN, VNN1, VWCE, AHI1, C9orf123, CCL4L1/CCL4L2, CENPA, CEP55, COMMD6, COX7B, CYP4F3, DTL, EP-STI1, ERAP2, GBP5, GLT25D2, GNLY, HINT1, HIST1H4A, HLA-DPB1, HMMR, HOXB2, IFI27, IFI44, IL18RAP, ITGB1BP1, KLRD1, KLRF1, PRSS23, PTER, RPL26, RPS27L, RSL24D1, SAMD9L, SCOC, SETD9, SUB1, TMEM126B, XAF1, ANXA3, CASP5, HERC5, IFI44, IFI44L, IFIT2, OAS3, PI3 et RSAD2.

3. Procédé selon l'une quelconque des revendications précédentes, dans lequel ledit sujet présente un risque de T1D conféré par HLA.

4. Procédé selon l'une quelconque des revendications précédentes, dans lequel ledit échantillon est un échantillon de tissu, un échantillon de sang total, ou un échantillon comprenant des cellules mononucléaires périphériques.

5. Utilisation d'un kit dans un procédé selon l'une quelconque des revendications 1 à 4, ledit kit comprenant un ou plusieurs agents d'essai capables de détecter le niveau d'expression de C17orf97 dans un échantillon biologique obtenu d'un sujet dont le risque de développer un T1D doit être déterminé.

6. Utilisation selon la revendication 5, dans laquelle ledit kit comprend en outre un ou plusieurs agents d'essai capables de détecter le niveau d'expression d'un ou plusieurs gènes choisis dans le groupe constitué de ADM, ALPL, ASGR2, BCL6, C19orf59, C3AR1, C4BPA, C5orf4, CACNG6, CBS, CCL8, CEACAM1, CLC, CNN2, CPPED1, CREB5, CTTN, CYSTM1, DCAF12, DEFA1, DSC2, DYSF, EMR3, EPB42, EPB49, FAM129A, FAM46C, FOLR3, GMPR, GPR84, HBD, HLA-C, IFIT3, IFITM3, IGF2BP2, IRF5, KIAA1324, KRT1, LILRB3, LRG1, MAFB, MBNL3, MMP9, MX1, MYOF, NFIX, NPCDR1, NPRL3, OAS1, OLIG2, OSBP2, PHOSPHO1, PMP22, PROK2, PRSS33, RAB31, RBPMS2, RFX2, RNASE2, ROPN1L, RRP12, SEC14L1, SELENBP1, SIRPB1, SLC22A4, SLC29A1, SLC4A1, SLC6A8, SMOX, SNCA, SORT1, TGM3, TMEM158, TNS1, TRIM58, TSHZ3, VCAN, VNN1, VWCE, AHI1, C9orf123, CCL4L1/CCL4L2, CENPA, CEP55, COMMD6, COX7B, CYP4F3, DTL, EP-STI1, ERAP2, GBP5, GLT25D2, GNLY, HINT1, HIST1H4A, HLA-DPB1, HMMR, HOXB2, IFI27, IFI44, IL18RAP, ITGB1BP1, KLRD1, KLRF1, PRSS23, PTER, RPL26, RPS27L, RSL24D1, SAMD9L, SCOC, SETD9, SUB1, TMEM126B, XAF1, ANXA3, CASP5, HERC5, IFI44, IFI44L, IFIT2, OAS3, PI3 et RSAD2.
